(19) Europäisches Patentamt
European Patent Office
Office européen des brevets

(11) Publication number: **0 429 775 A2**

(12) # EUROPEAN PATENT APPLICATION

(21) Application number: **90116910.2**

(22) Date of filing: **03.09.90**

(51) Int. Cl.5: **C07C 51/573**, C08G 73/10, C07C 63/313, C07C 63/331, C07C 65/24

(30) Priority: **01.09.89 JP 224445/89**

(43) Date of publication of application: **05.06.91 Bulletin 91/23**

(84) Designated Contracting States: **DE FR GB NL Bulletin**

(71) Applicant: **HITACHI, LTD.**
**6, Kanda Surugadai 4-chome**
**Chiyoda-ku, Tokyo 101(JP)**

Applicant: **Hitachi Chemical Co., Ltd.**
**1-1, Nishishinjuku 2-chome**
**Shinjuku-ku, Tokyo 160(JP)**

(72) Inventor: **Miwa, Takao**
**1-10-1, Kosuna-cho**
**Katsuta-shi, Ibaraki-ken(JP)**
Inventor: **Ikeda, Takayoshi**
**1161-3, Uchijuku, Tohkai-mura**
**Naka-gun, Ibaraki-ken(JP)**
Inventor: **Numata, Shunichi**
**3-1-15, Miyata-cho**
**Hitachi-shi, Ibaraki-ken(JP)**
Inventor: **Fujisaki, Koji**
**2-26-19, Higashiohnuma-cho**
**Hitachi-shi, Ibaraki-ken(JP)**
Inventor: **Shimanoki, Hisae**
**1-21-5, Ishinazaka-cho**
**Hitachi-shi, Ibaraki-ken(JP)**

(74) Representative: **Patentanwälte Beetz sen. - Beetz jun. Timpe - Siegfried - Schmitt-Fumian- Mayr**
**Steinsdorfstrasse 10**
**W-8000 München 22(DE)**

(54) **Carboxylic acid anhydride complexes and their preparation and use.**

(57) The invention relates to carboxylic acid anhydride complexes and particularly to carboxylic acid dianhydride complexes which are characterized by high hydrolysis and thermal stability and high solubility in organic solvents. They are prepared by reaction of electron donor compound having a donor number of at least 20 and being free from active hydrogen atoms in the molecule and a carboxylic acid anhydride compound. The invention relates also to compositions, varnishes and resins containing these complexes, and their use, particularly for the preparation of polyamic acides and polyimides.

EP 0 429 775 A2

# CARBOXYLIC ACID ANHYDRIDE COMPLEXES AND THEIR PREPARATION AND USE.

The present invention relates to carboxylic acid anhydride complexes and methods for their preparation, and to the composition etc. containing these complexes.

Carboxylic acid anhydrides have superior characteristics and are widely used, for example as cross-linking agents, and as monomers for the synthesis of polymers. However, acid anhydrides have a strong reactivity in general, and precautionary measures are required for storage and use. Especially, it is a big problem in the industry that carboxylic acid anhydrides react with moisture in the air and lose their reactivity. For example, carboxylic anhydrides have superior characteristics as hardeners for epoxy resins, however, their applicability is rather restricted because of their easy hydrolysability.

In the case of the synthesis of polyamic acids etc., hydrolysis of the carboxylic acid anhydrides causes a reduction of the monomer reactivity, and makes it difficult to produce a higher degree of polymerization. Hitherto, according to such unstability, the use of carboxylic acid anhydrides has been restricted although they possess superior characteristics. Therefore, a special treatment such as storage under dry conditions, reactivation by heating immediately before use, dehydration of a solvent, etc. are required heretofore to cope with the unstability of carboxylic acid anhydrides.

In the case of the synthesis of polyamic acids, etc., the degree of polymerization changes greatly depending on the equivalent ratio of the acid anhydride monomer and a reactant monomer such as an amine. That is, the molecular mass increases to infinity theoretically at the exact equivalent ratio and decreases sharply for equivalent ratios deviating from the equivalence. Accordingly, the molecular mass of the polymers can be controlled easily by controlling the equivalent ratio. A solution of an oligomer having a low molecular mass is easy in handling because of its low viscosity even at a high concentration. However, films made thereof and having a small molecular mass have insufficient mechanical strength.

On the other hand, solutions of polymers having a large molecular mass generally yield strong films, but the solutions are problematic in that a slight increase of the concentration of the solution causes a sharp increase of the viscosity so that solutions with too a high concentration cannot be practically used.

The solubility of the carboxylic acid anhydrides is generally very poor; for instance, acid anhydrides are only very slightly soluble even e.g. in N-methylpyrrolidon which is recognized as having the most preferable solubilization power, and the poor solubility is a great problem in the use of acid anhydrides; however, there have not been any suitable means available to solve this problem.

At present, solutions of polymers having a large molecular mass which can be easily handled even with high concentrations and also having a superior heat resistance, mechanical strength, and resistance to chemicals after curing, are needed widely. For example, for the polyimide production, oligoamic acids of small molecular mass, and imide oligomers and isoimide oligomers both of which have good solubility, have been used for this purpose. According to these methods, it is possible to use high concentration-low viscosity solutions. The use of oligomer solutions is preferred because it is easy to produce coatings, e.g. by spin coating etc., and superior coating films can be obtained by causing a reaction at the reactive end groups of the oligomer by heat treatment to get finally a large molecular mass polymer. As the reactive end groups, partially esterified acid anhydrides, ethynyl groups, vinyl groups, and biphenylene groups etc. have been used. For instance, electronic devices manufactured with oligomers having vinyl groups or acetylene groups as end groups of the molecules are disclosed in JP-A-60-120723.

A complex crystal of pyromellitic acid (PMDA) and N,N-dimethylacetamide (DMAC) in a molar ratio of 1:1 is described in Journal of Polymer Science, Part II, vol. 1, pp. 3135-3150. However, there is no description of the properties of the complex and no indication that it may be converted into a polyimide.

It is the object of the present invention to provide new carboxylic acid anhydride complexes, methods of producing same, and their use. The invention shall include polyamic acids and reaction products of carboxylic acid anhydride complexes with diamines, and corresponding complexes from which polyimides can be made. These products shall be provided in the form of compositions, varnishes, films, coatings, etc.

The object also comprises a method to produce polyimide layers which may be applied for the production of electronic devices, using the acid anhydride complexes and/or the polyamic acid complexes indicated above.

The above object s achieved according to the claims. The dependent claims relate to preferred embodiments.

The concept of the present invention solves the problems of the storage instability of carboxylic acid anhydrides and particularly the problem of the control of the molecular mass of polyamic acids regardless of the strong reactivity of organic compounds such as amines, and also the problem of the poor solubility of uncomplexed carboxylic acid anhydrides.

The complexes of the present invention can be utilized for the stabilization of acid anhydride compounds during storage, for the improvement of the solubility of acid anhydride compounds in organic solvents, for the viscosity control of polyamic acids, for processes and technologies where acid anhydrides are used, such as in manufacturing processes of electronic devices for producing varnishes, films, fibers etc.

The complexes of carboxylic acid anhydrides of the present invention comprise a carbonyl carbon atom with a controlled electrophilic reactivity.

The carboxylic acid anhydride complexes of the present invention have the general formula I,

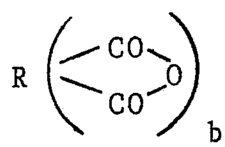 · cB (I),

wherein

R is the organic residue of the respective carboxylic acid anhydride, which may be derived from a dicarboxylic acid or a polycarboxylic acid,

B is an organic or inorganic electron donor compound forming a coordinate bond with an anhydride group, having a donor number of at least 20 and prefer ably of at least 25 and being free from active hydrogen atoms in its molecule in order to prevent cleavage of the anhydride ring,

b is an integer $\geqq 1$,

and

c is an integer $0 < c \leqq b$.

According to a preferred embodiment, the carboxylic acid dianhydride complexes have the general formula II,

$$Ar \cdot aB \qquad (II),$$

wherein

Ar is an organic carboxylic acid dianhydride,

B is an organic or inorganic electron donor compound forming a coordinate bond with an anhydride group and having a donor number of at least 20 and preferably at least 25 and being free from active hydrogen atoms in its molecule,

and

a is defined as $1 \leqq a \leqq 2$.

In accordance with another preferred embodiment, the complexes have the general formula III,

$$Ar' \cdot a'B \qquad (III),$$

wherein

Ar' is an oligomeric reaction product of an organic carboxylic acid dianhydride with an organic diamine, having at least one carboxylic acid anhydride group at an end of the molecule,

B is as defined above,

and

a' is defined as $a' \geqq 1$.

Preferred complexes have the general formula IV,

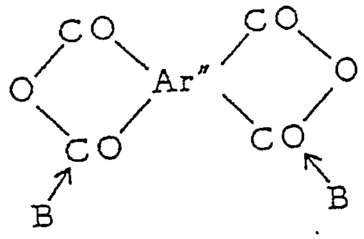

(IV),

wherein

Ar" is a residual group of a carboxylic acid dianhydride, which may also be an oligomeric group, e.g. resulting from a polyamic acid formation reaction,
and

B is as defined above.

Further preferred complexes according to the present invention have the general formula V,

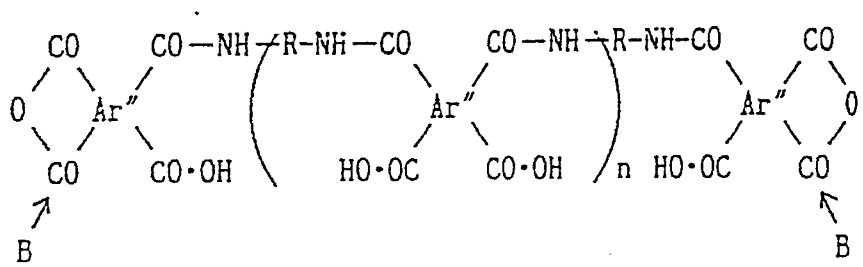

(V),

wherein

Ar" is a residual group of a carboxylic acid dianhydride,

R is an organic residual group of a diamine,
and

n is zero or an integer of at most 20.

The general method of the present invention for preparing the carboxylic acid anhydride complexes as defined above is characterized by contacting a compound comprising one or more carboxylic acid anhydride groups, particularly of the general structure

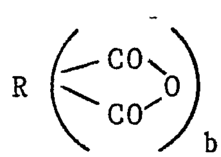

as defined above, and particularly a carboxylic acid dianhydride Ar or

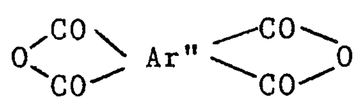

as defined above with an electron donor compound B, where R, b, Ar, Ar" and B and the respective relative amounts of the components are defined as before.

The carboxylic acid anhydride/dianhydride is preferably brought into contact with the electron donor compound B in the gaseous state or in the vapor phase. Alternatively, it may be dispersed in a solvent in which it is insoluble, but the respective complex is soluble, and the elctron donor compound B is added thereto, preferably under heating.

The compositions of the present invention are preferably liquid compositions, comprising one or more complexes as defined above, and a solvent for the complex(es).

The invention includes also resin compositions, comprising one or more complexes as defined above, and one or more other resins, particularly thermosetting resins.

According to the general concept of this invention, any carboxylic acid anhydride may be used for complexing. Typical examples, which are preferably used for preparing polyamic acids and polyimides, are carboxylic acid dianhydride used in the present invention is an carboxylic acid dianhydride such as pyromellitic dianhydride (PMDA), benzophenone tetracarboxylic dianhydride (BTDA), 3,3',4,4'-biphenyl tetracarboxylic dianhydride (s-BPDA), 3,3',4,4'-biphenylsulfone tetracarboxylic dianhydride (DSDA), 2,2-bis-(3,4-dicarboxyphenyl) hexafluoropropane tetracarboxylic dianhydride (6FDA), methylpyromellitic dianhydride, dimethylpyromellitic acid dianhydride, trifluoromethylpyromellitic acid dianhydride, bis-(trifluoromethyl)-pyromellitic acid dianhydride, 3,3',4,4'-oxydiphenylene tetracarboxylic acid dianhydride, etc. The acid anhydrides in general, and also the acid dianhydrides mentioned above may be used separately or together in the form of mixtures, as required.

In the present invention, the term "donor number" is defined in accordance with the definition given on pages 21-29 of the book entitled "Youeki Han-nou no Bunshikan Sougosayou" (published 1986 by Gakkai Shuppan Center, translated by Ohtaki Hitoshi et al.), translated from the monograph "Donor-Acceptor Approach to Molecular Interaction" (V. Gutmann, 1978). That is, the donor number is defined as the value of molar enthalpy of the reaction between $10^{-3}$M $SbCl_5$ in dichloroethane which is selected as a standard acceptor, and a donor (D, or B, resp.).

Basic organic substances that is electron donors which may be used in the complex formation reaction are substances having a donor number of at least 20. Especially, in the case of using a basic organic substance having a donor number of at least 25, the production of a complex is easier in the reaction mentioned above. Typical examples are tetrahydrofuran (THF), trimethylphosphate (TMP), tributylphosphate (TBP) etc. Especially effective substances having a donor number of at least 25 are e.g. dimethylformamide (DMF), N-methylpyrrolidon (NMP), N-dimethylacetamide (DMA), dimethylsulfoxide (DMSO), N-diethylformamide (DEF), N-diethylacetamide (DEA), N-methylacetamide, pyridine (PY), hexamethylphosphoric acid triamide (HMPA), tetramethylurea, triethylamine (TEA), etc. In addition, e.g. $\gamma$-propiolactam and $\epsilon$-caprolactam, etc., are suitable donor substances. The electron donors may be used separately or together in the form of mixtures.

According to the general concept of the present invention, any electron donor fulfilling the above donor strength criterian may be used.

Because of the main interaction (coordinate band formation) between an carboxylic acid anhydride group and an electron donor group of an electron donor (complexant) as defined above, the theoretical ratio of anhydride groups and donor groups in the complexes of the present invention is 1:1 (equivalent ratio).

The same applies for complexed groups in a molecule which may contain further, non-complexed anhydride groups; also in this case, the theoretical ratio of anhydride and donor groups forming components of a respective complex or a complex structure of a molecule, is 1:1.

Depending on the kind of anhydride compound (compound comprising one, two or more anhydride groups in the molecule) and of the kind of the electron donor compound (compound comprising one, two or more interacting donor groups in the molecule), the molar ratio of anhydride and donor compounds may be different from the equivalent ratio.

The complex formation utilized according to the present invention is an equilibrium reaction in principle, the complex stability constants resulting therefrom depending on the kind of anhydride and donor molecules, particularly the neighbour groups of the interacting groups.

For this reason, the resulting product, particularly in solutions, may be a mixture of complex and free, non-complexed anhydride and/or donor.

The reactivity of acid anhydrides is generally determined by hydrolysis to the corresponding carboxylic acid or by means of esterification with an alcohol. However, the acid anhydride complexes of the present invention are very stable, and require generally heating up to nearly 200 °C for reactivation. Therefore, these methods can be used only in extremely limited cases.

The carboxylic acid anhydride complexes of the present invention are able to recover their activity as an acid anhydride easily with heating. Moreover, the complex is extremely stable at moderate temperatures which is depending on the kind of complex, generally below 150 °C, in some cases below 80 °C.

As a result of the complex formation and the electron interaction with the electron donor, the degree of polarization $\delta^+$ of the carbonyl carbon in the acid anhydride is decreased. The reactivity of the complex for electrophilic reactions such as hydrolysis, acylation etc., is determined by the degree of polarization $\delta^+$.

The case of complexation is different from the case where the reactivity of an acid anhydride is lowered by hydrolysis or esterification, and an aimed reaction is easily caused by heating. Therefore, the method of anhydride stabilization of the present invention does not harm the reactivity of an acid anhydride.

Accordingly, the method of this invention for producing stable complexes is extremely superior for stabilizing acid anhydrides.

Additionally, as a result of investigations on the stability of such complexes, it has been found that donors having a donor number of at least 25, such as N-methylpyrrolidon, dimethylsulfoxide, triethylamine, pyridine, etc., give complexes of specially good stability.

Tetracarboxylic dianhydrides have generally a very poor solubility in organic solvents in a not complexed form, and are only slightly soluble in organic solvents such as N-methylpyrrolidon etc. Therefore, the solvents which could be used therefor were extremely limited. On the other hand, the solubility of acid anhydride complexes according to the present invention is significantly improved, and the complexes are easily soluble, e.g. in tetrahydrofuran which could practically not be used hitherto for acid anhydrides. Thus, the acid anhydride complexes obtained according to the present invention are very effective for improving the solubility. The acid anhydride complexes of the present invention are electron donor-acceptor complexes with the electron donor as a complex former, which combines to one of the carbon atoms of the carbonyl groups of the acid anhydride by a coordinate bond, as may be seen from the following schematic reaction equation (1) which relates to a tetracarboxylic acid dianhydride (bifunctional with respect to complex formation) and a monofunctional donor B:

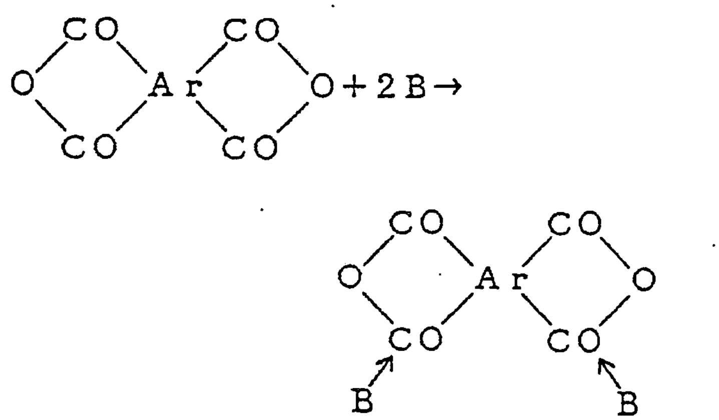

···(1)

If there are too many molecules of electron donor around one acid anhydride molecule, for example, in the case where an acid anhydride is dissolved in an electron donor solvent, electron donor molecules surround the molecules of the acid anhydride and cause solvation which prevents the formation of a coordinate bond. In the case described above, the formation of the acid anhydride complex is not completed, and, even if the solution is heated, the molar ratio of the acid anhydride and the electron donor in a complex is nearly 1:1 at most, and the yield is less than 30 %.

To make the molar ratio smaller than 1:1, it is preferred to make the acid anhydride contact with the electron donor in the gaseous state. For instance, contacting an acid anhydride with a vaporized electron donor causes a sufficient reaction without solvation. Alternatively, the complex formation reaction may be effected by dropwise adding a small amount of the electron donor to the solid acid anhydride, preferably as a powder, with agitation and under heating. Another alternative is dropwise addition of the electron donor under heating to a liquid in which the acid anhydride is dispersed in a solvent which dissolves the complex but not the acid anhydride itself.

In any case, particularly for dianhydrides, a complex having a molar ratio of 1:2 of acid anhydride and electron donor is most stable against hydrolysis and is superior in solubility in organic solvents. Depending on the reaction conditions, complexes having a molar ratio slightly higher than 1:2 are obtainable. It is clear that even a complex having a combination ratio slightly higher than 1:2 has better stability and solubility than a complex having a ratio of 1:1.

It is also possible to prepare complexes with oligomers, e.g. by a reaction of a complex according to the present invention with a diamine compound, e.g. in accordance with the following schematic reaction equation (2):

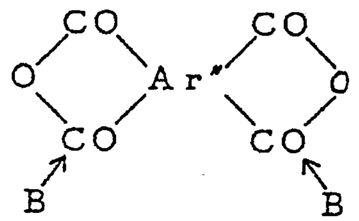 + $H_2N-R-NH_2$ →

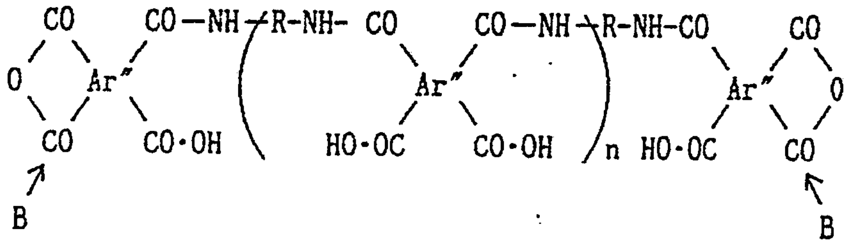 ···(2)

$(20 \geqq n \geqq 0$, especially $10 \geqq n \geqq 0)$.

The acid dianhydride complexes of the present invention may be present in the form of Ar•2B, and of mixtures of Ar•2B and Ar•B. By selecting the reaction conditions, the mixing ratio of Ar•2B and Ar•B can be changed; reaction conditions which enable Ar and B to react sufficiently, increase the Ar•2B content. It is preferable to make the coefficient a in Ar•aB to be at least 1.5 for obtaining best hydrolysis stability and solubility of the complex. Values of a of at least 1.5 in Ar•aB are obtainable by one-step reaction or by suitable mixing of Ar•2B and Ar•B which are made separately.

According to the superior characteristics of the complexes, various applications are possible. Polyimides, important substances in industry, are generally obtained by coating a polyamic acid and subsequent heating and hardening. In the synthesis of polyamic acids, which are precursors of polyimides, complexes of the present invention are applicable to produce varnishes which are easy to handle even at high concentrations. The resulting polyimides have the same superior mechanical characteristics, heat resistance, and resistance to chemicals after hardening as in the case of using conventional high molecular mass polyamic acids. That is, e.g. a varnish containing an acid anhydride complex having a monomer or an oligomer structure in the molecule and an equivalent diamine compound such as e.g. p-phenylene diamine (PDA) polymerizes to a high molecular mass substance by heating which causes a reaction between the acid anhydride complex and the amine compound.

The viscosity of the varnish in the case according to the invention is far lower than that of a conventional polyamic acid varnish of the same concentration, because the viscosity of a high polymer solution is proportional to the third power of the molecular mass. Therefore, a highly concentrated solution can be practically applied wherein a varnish containing the complex mentioned above is used. Moreover, the varnish after hardening becomes a high molecular mass substance which has the same superior mechanical characteristics, heat resistance, and resistance to chemicals as in the case of using a conventional polyamic acid varnish. And of course, it is possible to adjust easily the viscosity of a varnish by changing the content of the complex in the acid anhydride.

It is well known that polyimides having a rod-like structure have a low thermal expansion coefficient. Therefore, by using a monomer which will produce a polyimide having a rod-like structure, it is possible to obtain a polyimide type resin precursor which has superior properties for producing flat insulating films leading to only a small thermal stress. It is easily assumed that, if the polyimide obtained finally therefrom has a rigid, rod-like structure, it has a low thermal expansion coefficient. And, as the low thermal expansion property comes from the structure of skeleton of the main chain, it is obvious that an improvement of the property is possible by introducing substituents selected from alkyl, fluorinated alkyl, alkoxyl, fluorinated alkoxyl, aryl, halogen etc., into the monomers.

By copolymerization with other diamine compounds and acid dianhydrides, other improvements are possible, such as producing a more flexible polyimide by copolymerization with a polymer having a 'soft' structure, such as 3,3',4,4'-benzophenone etc., within the range of not losing the property of low thermal expansion, or improving the adhesiveness by copolymerization with a substance such as 1,3-bis(3,4-dicarboxy[1.2.2] bicyclo)-tetramethyldisiloxane dianhydride etc.

And it is also possible to control ability of wet etching, which is important in the manufacturing process of large scale integrated circuits (LSI), by copolycondensation of pyromellitic acid dianhydride and 3,3'4,4-biphenyltetracarboxylic acid dianhydride.

The acid anhydride complexes of the present invention are compounds in which the electrophilic reactivity of the acid dianhydride is controlled by formation of a complex with the basic organic compound which is an electron donor having a donor number of at least 20 and being free from active hydrogen; it is characteristic that they quickly recover the reactivity by heating. By utilizing the characteristics mentioned above, it is possible to obtain acid anhydride complexes which are stable against hydrolysis, and varnishes which yield polyimides which are high molecular mass substance by heating, and which reveal superior characteristics although they have low viscosity and a high concentration of low molecular mass substance in the varnish state.

The acid anhydride complexes of the present invention can also be used as curing agents for epoxy resins, etc., by utilizing the property of becoming reactive by heating.

A study on the thermal decomposition temperature of complexes obtained according to the present invention gave the results shown in Table 1. The data in Table 1 show that it is possible to obtain acid anhydride complexes having the property to be active at a desired temperature by suitable selection of an electron donor.

On the other hand, the thermal decomposition temperature of s-BPA, which is obtained by hydrolysis of s-BPDA, is 173 °C, and its endothermic peak is 257 °C.

Besides, the melting points of the acid anhydrides are as follows:
s-BPDA: 294 °C; BTDA: 230 °C; PMDA: 228 °C; 6FDA: 241 °C; DSDA: 280 °C; OPDA: 218 °C.

The boiling points of the electron donors are as follows:
NMP: 203 °C; DMSO: 185 °C; PY: 115 °C; TEA: 89 °C.

Table 1

| Acid anhydride | Complex former | Endothermic peak (°C) |
|---|---|---|
| s-BPDA | NMP | 104 |
| 〃 | DMSO | 126 |
| 〃 | PY | 203 |
| 〃 | TEA | 239 |
| BTDA | NMP | 102 |
| 〃 | DMSO | 222 |
| 〃 | PY | 190 |
| 〃 | TEA | 254 |
| PMDA | NMP | 155 |
| 〃 | DMSO | 231 |
| 〃 | TEA | 155 |
| 6FDA | NMP | 208 |
| 〃 | DMSO | 210 |
| 〃 | PY | 221 |
| 〃 | TEA | 257 |
| OPDA | DMSO | 210 |

In the following, the invention will be explained by way of examples with reference to the drawings.

Fig. 1 is the proton Nuclear Magnetic Resonance (NMR) spectrum ($^1H$-NMR) of s-BPDA,
Fig. 2 is the proton NMR spectrum of s-BPDA/PY complex;
Fig. 3 is the proton NMR spectrum of s-BPDA/NMP complex;
Fig. 4 is the proton NMR spectrum of PMDA;
Fig. 5 is the proton NMR spectrum of PMDA/TEA complex;
Fig. 6 is the proton NMR spectrum of 6FDA;
Fig. 7 is the proton NMR spectrum of 6FDA/PY complex;
Fig. 8 is the proton NMR spectrum of a bisimide compound;
Fig. 9 (a) is a liquid chromatogram of s-BPDA;
Fig. 9 (b) is a liquid chromatogram of the compound produced by reaction of s-BPDA with ϵ-caprolactam in 10 hours at 180 °C - 200 °C;
Fig. 9 (c) is a liquid chromatogram of the compound produced by reaction of s-BPDA with ϵ-caprolactam in 24 hours at 180 °C - 200 °C;
Fig. 10 is the proton NMR spectrum of s-BPDA/DMSO complex immediately after synthesizing;
Fig. 11 is the proton NMR spectrum of s-BPDA/DMSO complex after 18 hours from synthesis under moisture conditions;
Fig. 12 is a perspective illustration showing the structure of the evaluation pattern to be used for the measurement of flatness of a polyimide film produced from an acid dianhydride by the process of the present invention, and
Fig. 13 is a cross sectional view showing the definition of the flatness of a polyimide film.

**Example 1**

An acid anhydride complex having a molar ratio of 1:2 of s-BPDA:PY was obtained by reaction of s-BPDA with saturated PY vapour in 40 hours at 100 °C. The nuclear magnetic resonance spectrum of s-BPDA is shown in Fig. 1, and the nuclear magnetic resonance spectrum of the obtained complex is shown in Fig. 2. The solvent used at the measurement was DMF-$d_7$. After heating of the acid anhydride complex in DMSO for 2 hours at 120 °C, the yield of the acid anhydride complex was 50 %. And after heating of the acid anhydride complex in PY 1 hour at 100 °C, the yield of the acid anhydride complex was 60 %.

**Example 2**

An acid anhydride complex of s-BPDA:NMP having a molar ratio of 1:2 was obtained nearly quantitatively by reaction of s-BPDA with saturated NMP vapour in 40 hours at 200 °C. The nuclear magnetic resonance spectrum of the obtained complex is shown in Fig. 3.

**Example 3**

An acid anhydride complex of s-BPDA:TEA having a molar ratio of 1:2 was obtained by reaction of s-BPDA with saturated TEA vapour in 40 hours at 80 °C.

**Example 4**

An acid anhydride complex of PMDA:PY having a molar ratio of 1:2 was obtained by reaction of PMDA with saturated PY vapour in 40 hours at 100 °C.

**Example 5**

An acid anhydride complex of PMDA:TEA having a molar ratio of 1:2 was obtained nearly quantitatively by reaction of PMDA with saturated TEA vapour in 40 hours at 80 °C. The nuclear magnetic resonance spectrum of the obtained PMDA complex is shown in Fig. 5.

**Example 6**

An acid anhydride complex of PMDA:NMP having a molar ratio of 1:2 was obtained nearly quantitatively by reaction of PMDA with saturated NMP vapour in 40 hours at 200 °C.

**Example 7**

An acid anhydride complex of BTDA:PY having a molar ratio of 1:2 was obtained nearly quantitatively by reaction of BTDA with saturated PY vapour in 40 hours at 100 °C. After heating the acid anhydride complex in DMSO 2 hours at 120 °C, the yield of the acid anhydride complex was 75 %.

**Example 8**

An acid anhydride complex of 6FDA:PY having a combination ratio of 1:2 was obtained nearly quantitatively by reaction of 6FDA with saturated PY vapour in 40 hours at 100 °C. The nuclear magnetic resonance spectrum of the obtained 6FDA complex is shown in Fig. 7.

**Example 9**

An acid anhydride complex of 6FDA:TEA having a molar ratio of 1:2 was obtained nearly quantitatively by reaction with 6FDA and saturated TEA vapour in 40 hours at 80 °C.

**Example 10**

A reaction was carried out by dropwise adding NMP in 3 hours to 60 g of s-BPDA powder with stirring at 80 to 120 °C in an inert atmosphere, and a brown powder was obtained. The nuclear magnetic resonance spectrum of s-BPDA and the reaction product were measured. The formation of an acid anhydride complex was confirmed by observation of a peak shifting to lower magnetic field.

**Example 11**

A reaction was carried out by dropwise adding DSMO in 3 hours to 60 g of s-BPDA powder with stirring at 80 to 120 °C in an inert atmosphere, and a brown powder was obtained. The nuclear magnetic resonance spectrum of the reaction product was measured. The formation of an acid anhydride complex was confirmed by observation of a peak shifting to lower magnetic field.

**Example 12**

A reaction was carried out by dropwise adding THF and γ-propiolactam in 3 hours to 60 g of s-BPDA powder with stirring at 80 to 120 °C in an inert atmosphere, and a brown powder was obtained. The nuclear magnetic resonance spectrum of the reaction product was measured. The formation of an acid anhydride complex was confirmed by observation of a peak shifting to lower magnetic field.

In other experiments with use of lactams having different ring size from 5 to 10 atoms under the same condition, the formation of acid anhydride complexes was confirmed by observation of the nuclear magnetic resonance spectra. Also in the case of using N-methylacetamide, which is a same secondary amide as a lactam, the formation of a complex was also confirmed.

**Example 13**

A mixture of 240 g of THF and 6 g of s-BPDA was heated 1 to 3 hours with PY in an inert atmosphere, and a yellow transparent solution was obtained. By adding the yellow solution to 20 times the volume of n-hexane, a pale yellow substance was precipitated. The precipitate was separated from the liquid by filtration and was dried 12 hours at 60 °C in vacuum. 7.2 g of powder were obtained. The formation of a complex was confirmed by observation of the same peak shifting to lower magnetic field as shown in Fig. 2 in the nuclear magnetic resonance spectrum of the powder.

**Example 14**

A mixture of 240 g of caprolactam, 6 g of s-BPDA and γ-propiolactam was heated 1 to 3 hours in an inert atmosphere, and a yellow transparent solution of a complex was obtained. The formation of a complex was confirmed by observation of the same peak shifting to lower magnetic field as shown in Fig.2 in the nuclear magnetic resonance spectrum of the solution.

**Example 15**

The formation of a complex in the reaction product obtained by the same experiment as Example 10 except that BTDA was used instead of s-BPDA was confirmed by observation of a peak shifting to lower magnetic field in the nuclear magnetic resonance spectrum of the reaction product and BTDA.

**Example 16**

The formation of a complex in the reaction product obtained by the same experiment as Example 10 except that PMDA was used instead of s-BPDA was confirmed by observation of a peak shifting to lower magnetic field in the nuclear magnetic resonance spectrum of the reaction product and PMDA.

**Comparative Example 1**

A mixture of 240 g of NMP, 60 g of s-BPDA and ε-caprolactam was heated 36 hours at 180 to 200 °C in an inert atmosphere, and a brown solution was obtained. The nuclear magnetic resonance spectrum of the solution is shown in Fig. 8. Formation of any complex is not observed, but the quantitative formation of bisimide compound was confirmed. The time depending change of the yield of the product was measured by a chromatographic method, and the results are shown in Figs. 9 (a) to 9 (c). The reaction was very slow, and the formation of the bisimide carboxylic acid was scarcely observed after a reaction time of 1 to 3 hours.

**Example 17**

A mixture of s-BPDA and a complex comprising s-BPDA and DMSO was dissolved in DMSO containing water. The changing of the composition was observed by measurement of the nuclear magnetic resonance spectrum. The results are shown in Figs. 10 and 11. It was revealed that s-BPDA was hydrolyzed as the peaks of s-BPDA which were observed at the time of dissolution had been disappeared after 18 hours. On the other hand, the peaks of s-BPDA complex were observed even after 18 hours without any change. The results of the experiment mentioned above revealed that the formation of a complex lowers the hydrolyzability and significantly increases the stabilizing of s-BPDA.

**Example 18**

A reaction was carried out by slowly adding an equivalent of p-PDA to a solution of s-BPDA complex which was obtained in Example 1 with stirring and ice-cooling. The viscosity of the varnish after a reaction of 3 hours with stirring was 8 poises at a solid content of 30 % by mass.

**Example 19**

The same experiment as in Example 9 except that an equivalent of DDE was used instead of p-PDA was carried out. The viscosity of the varnish obtained was 15 poises at a solid content of 30 % by mass.

**Example 20**

A reaction was carried out by slowly adding an equivalent of p-PDA to a solution of the complex obtained in Example 4 in NMP with stirring. The viscosity of the varnish after a reaction of further 3 hours with stirring was 42 poises at a solid content of 30 % by mass.

**Comparative Example 2**

A solution was prepared by dissolving 22 g of p-PDA in 240 g of NMP with stirring. A reaction was carried out by adding an equivalent (60 g) of s-BPDA to the solution with slowly stirring by means of a stirrer connected to a motor and ice-cooling in an inert atmosphere. The viscosity of the reaction mixture was increased as the addition of the solution was going on, and finally, at the time when the solution was completely added, stirring became impossible because of the increased viscosity.

**Comparative Example 3**

The same reaction as in Comparative Example 2 was carried out except that an equivalent (41 g) of DDE was used instead of p-PDA. It became impossible to stir the reaction mixture half way of the reaction.

**Example 21**

The varnish obtained in Example 17 was applied on the surface of a glass substrate with an applicator and was dried one hour at 100 °C and hardened by heating up to 400 °C at a rate of 200 °C/hour and kept 10 minutes at 400 °C. The obtained film was cut into a test piece of 5 mm x 50 mm, and its mechanical strength was measured. The rupture strength of the film was 36 kg/mm$^2$, and the rupture elongation was 25 %. The durability temperature defined as the temperature at which a 3 % loss in mass occurs in 100 minutes was 520 °C.

**Example 22**

The varnish synthesized in Example 19 was hardened at 350 °C as the final hardening temperature, and the mechanical strength of the film was measured. The rupture strength was 28 kg/mm$^2$, and the rupture elongation was 52 %. The durability temperature was 491 °C defined as in Example 21.

**Example 23**

A film was prepared from the varnish prepared in Example 20 by the same process as in Example 12, and the mechanical strength was measured. The rupture strength was 41 kg/mm$^2$, and the rupture elongation was 22 %. The durability temperature was 517 °C defined as in Example 20.

**Comparative Example 4**

A reaction was carried out by slowly adding 2/3 of an equivalent (54.4 g) of s-BPDA to a solution of 30 g of p-PDA in 200 g of NMP. The reaction was carried on further 5 hours after the s-BPDA was added, and a dense green solution was obtained. By adding 27.2 g of phthalic acid anhydride to the solution so as to make the ratio of amine and acid anhydride equivalent and carrying on the reaction for further 5 hours, a yellow transparent oligomer varnish having a viscosity of 25 poises was obtained. The varnish was hardened by the same process as in Example 20. In the hardening process, a large number of cracks were generated on the surface of the hardened body, and no film could be obtained. The measurement of the mechanical strength was impossible.

**Comparative Example 5**

A reaction was carried out by slowly adding 2/3 of an equivalent (31.3 g) of PMDA to a solution of 50 g of DDE in 200 g of NMP. The reaction was carried out further 5 hours after the PMDA was added, and a dense green solution was obtained. By adding 21.6 g of phthalic anhydride to the solution so as to make the ratio of amine and acid anhydride equivalent and carrying on the reaction for further 5 hours, a yellow transparent oligomer varnish having a viscosity of 19 poises was obtained. The varnish was hardened by the same process as in Example 20. In the hardening process, a large number of cracks were generated on the surface of the hardened body as in Comparative Example 6, and no film could be obtained. The measurement of the mechanical strength was impossible.

**Comparative Example 6**

A half-esterified solution was synthesized by reaction of 60 g of s-BPDA and 2 equivalents of ethanol in 200 g of NMP in 2 hours at 100 °C. The solution was cooled down to room temperature and was added with an equivalent of p-PDA to s-BPDA. By dissolving the additives with stirring, a half-ester varnish which could be polymerized in a hardening process was obtained. The viscosity of the varnish was 1.8 poises. In the same hardening process as in Comparative Example 5, a large number of cracks were generated on the surface of the hardened body, and no film could be obtained. The measurement of the mechanical strength was impossible.

**Comparative Example 7**

A varnish solution having a concentration of 40 mass-% and a viscosity of 42 poises was obtained by dissolving a resin having ethynyl groups at the terminal of the molecular chain in NMP. The varnish was hardened by the same process as in Example 21. The obtained film was too fragile to be measured for its mechanical strength.

**Example 24**

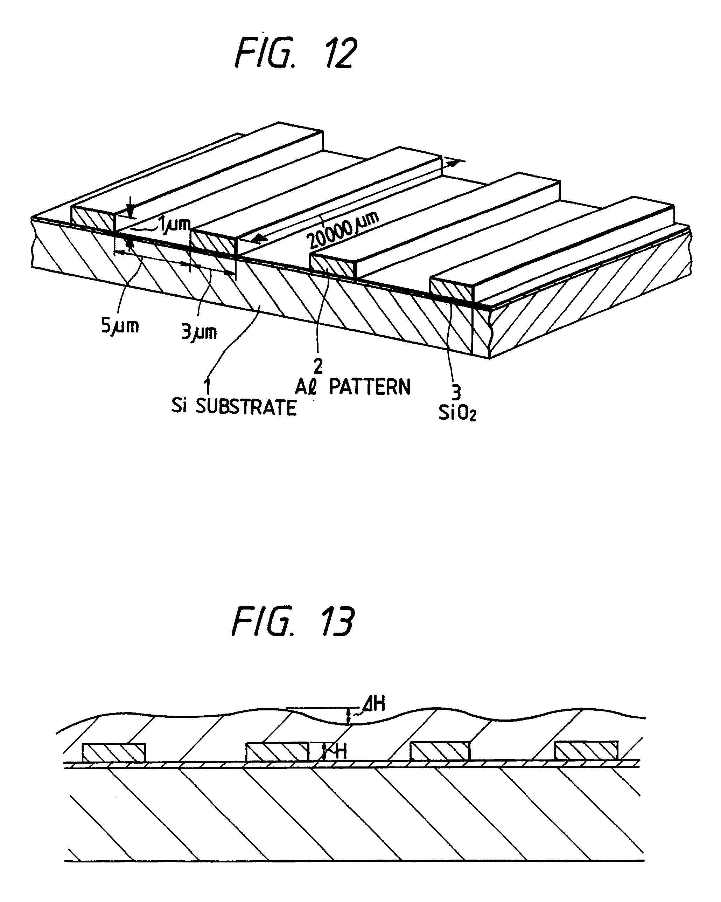

The result of the measurement of flatness of a film produced from the varnish synthesized in Example 17 by applying it on the surface of an aluminum pattern the structure of which is shown in Fig. 12, and hardening was 0.80. The flatness is defined by the following equation:

$$P = 1 - \frac{\Delta H}{H} \qquad \text{---(3)}$$

The more the value is closer to 1, the more it is preferred.

The symbols in equation (3) are defined in Fig. 13. The evaluation pattern to be used for the measurement of the flatness of a polyimide film is shown in Fig. 12. Using the pattern, the flatness of a film produced on the surface of the pattern was measured according to the definition shown in Fig. 13.

**Example 25**

The result of the measurement of flatness of a film produced from the varnish synthesized in Example 18 by applying it on the surface of an aluminum pattern the structure of which is shown in Fig. 12, and hardening was 0.83.

**Comparative Example 8**

A polyamic acid varnish having a concentration of 15 mass-% which was synthesized from p-PDA and s-BPDA by a conventional process was applied and hardened by the same process as in Example 25, and the flatness of the film was measured. The result was 0.44.

**Example 26**

A transparent brown solution of a complex was obtained by adding 6 g of BTDA to 240 g of THF and heating 1-3 hours with DMSO in an inert atmosphere. The formation of a complex was confirmed by observation of the nuclear magnetic resonance spectrum of the solution which was measured after the same treatment as in Example 13.

**Example 27**

A transparent brown solution of a complex was obtained by adding 6 g of BTDA and $\gamma$-propiolactam to 240 g of THF and heating 1-3 hours in an inert atmosphere. In the nuclear magnetic resonance spectrum measured after the same treatment as in Example 13, a peak shifting to lower magnetic field was observed, and the formation of a complex was confirmed. In the same experiment except that different ring sizes of lactams from 5 to 10 atoms were used, the formation of a complex was confirmed by the nuclear magnetic resonance spectrum. In the case of using N-methylacetamide, the formation of a complex was also confirmed.

**Example 28**

A transparent yellow solution was obtained by adding 6 g of BTDA to 240 g of THF and heating 1-3 hours with PY in an inert atmosphere. A yellow precipitate was obtained by adding the solution to 20 times the volume of n-hexane. After the same treatment as in Example 13, the nuclear magnetic resonance spectrum was measured. In the spectrum, a peak shifting to lower magnetic field was observed, and the

formation of a complex was confirmed.

**Example 29**

A transparent yellow solution of a complex was obtained by adding 6 g of BTDA and γ-propiolactam to 240 g of caprolactone and heating 1-3 hours in an inert atmosphere. After the same treatment as in Example 13, the nuclear magnetic resonance spectrum was measured. A peak shifting to lower magnetic field was observed, and the formation of a complex was confirmed.

**Comparative Example 9**

A brown solution was obtained by adding 6 g of BTDA and ε-caprolactam to 240 g of NMP and heating 36 hours at 180 to 200 °C in an inert atmosphere. According to the nuclear magnetic resonance spectrum of the solution, the formation of a complex was not observed, but quantitative yield of bisimide compound was confirmed. By measuring the time depending change of the yield of the product by a liquid chromatographic method, it was revealed that the reaction went on very slowly, and the formation of bisimide carboxylic acid was scarcely observed in a reaction time of 1-3 hours.

**Example 30**

A mixture of BTDA and a complex comprising BTDA and DMSO was dissolved in DMSO containing water, and the change of the solution was measured by the nuclear magnetic resonance spectrum. The result revealed that the peaks of BTDA which were observed at the moment of dissolving disappeared after 18 hours, and hydrolysis of BTDA had occured. On the other hand, the peaks of the complex including BTDA were observed even after 18 hours without any change, and it was confirmed that the formation of a complex occured lowering the hydrolyzability and giving a significant stabilization of BTDA.

**Example 31**

A transparent brown solution of a complex was obtained by adding 60 g of DSDA to 240 g of THF and heating 3 hours with NMP in an inert atmosphere. According to the nuclear magnetic resonance spectrum which was measured after the solution was treated by the same process as in Example 13, a peak shifting to lower magnetic field was observed, and the formation of a complex was confirmed.

**Example 32**

A transparent brown solution of a complex was obtained by adding 6 g of DSDA to 240 g of THF and heating 3 hours with DMSO in an inert atmosphere. According to the nuclear magnetic resonance spectrum which was measured after the solution was treated by the same process as in Example 13, a peak shifting to lower magnetic field was observed, and the formation of a complex was confirmed.

**Example 33**

After adding 60 g of DSDA and γ-propiolactam to 240 g of THF, the solution was heated 3 hours in an inert atmosphere. After the solution was treated by the same process as in Example 13, the nuclear magnetic resonance spectrum was measured. A peak shifting to lower magnetic field was observed in the spectrum, and the formation of a complex was confirmed. In the same experiment except that the ring size of the lactams was changed to 5-10 atoms, the formation of a complex was confirmed with the nuclear magnetic resonance spectrum. In the case of using N-methylacetamide which is a same secondary amine as the lactam, the formation of a complex was also confirmed.

**Example 34**

A transparent yellow solution was obtained by adding 6 g of DSDA to 240 g of THF and heating 3 hours with PY in an inert atmosphere. A pale yellow precipitate was obtained by adding the solution to 20 times the volume of n-hexane. After the precipitate was treated by the same process as in Example 4, the nuclear magnetic resonance spectrum was measured. A peak shifting to lower magnetic field was observed, and the formation of a complex was confirmed.

**Example 35**

A transparent yellow solution was obtained by adding 60 g of DSDA and γ-propiolactam to 240 g of caprolactone and heating 3 hours with γ-propiolactam in an inert atmosphere. After the precipitate was treated by the same process as in Example 4, the nuclear magnetic resonance spectrum was measured. A peak shifting to lower magnetic field was observed, and the formation of a complex was confirmed.

**Comparative Example 10**

A brown solution was obtained by adding 60 g of DSDA and ε-caprolactam to 240 g of NMP and heating 36 hours at 180 to 200 °C in an inert atmosphere. According to the nuclear magnetic resonance spectrum of the solution, the formation of a complex was not observed, but the quantitative formation of a bisimide carboxylic acid was confirmed. The reaction went on slowly, and the formation of bisimide carboxylic acid was scarcely observed in a reaction time of 1-3 hours.

**Example 36**

A mixture of DSDA and a complex of DSDA was dissolved in DMSO containing water, and the change of the solution was measured with the nuclear magnetic resonance spectrum. The peak of DSDA which was observed at the moment of dissolution disappeared after 18 hours, and hydrolysis of DSDA was confirmed. On the other hand, the peak of the complex of DSDA was observed even after 18 hours without any change. The result revealed that the formation of a complex occured lowering the hydrolyzability and giving a significant stabilization of DSDA.

**Example 37**

A transparent brown solution of a complex was obtained by adding 60 g of 6FDA to 240 g of NMP and heating 3 hours at 80 to 120 °C in an inert atmosphere. In the nuclear magnetic resonance spectrum of 6FDA and of the solution, a peak shifting to lower magnetic field was observed, and the formation of a complex was confirmed.

**Example 38**

A transparent brown solution of a complex was obtained by adding 6 g of 6FDA to 240 g of THF and heating 3 hours with DMSO in an inert atmosphere. After the solution was treated by the same process as in Example 4, the nuclear magnetic resonance spectrum was measured. A peak shifting to lower magnetic field was observed, and the formation of a complex was confirmed.

**Example 39**

A transparent brown solution of a complex was obtained by adding 60 g of 6FDA and γ-propiolactam to 240 g of THF and heating 3 hours in an inert atmosphere. After the solution was treated by the same process as in Example 4, the nuclear magnetic resonance spectrum was measured. The formation of a complex was confirmed by the observation of a peak shifting to lower magnetic field in the spectrum. In the same experiment except that the ring size of the lactam was changed to 5-10 atoms, the formation of a complex was confirmed with the nuclear magnetic resonance spectrum. In an experiment using N-methylacetamide which is same secondary amide as the lactam, the formation of a complex was also confirmed.

**Example 40**

A transparent yellow solution was obtained by adding 6 g of 6FDA to 240 g of THF and heating 3 hours in an inert atmosphere. According to the nuclear magnetic resonance spectrum measured after the solution was treated by the same process as in Example 4, a peak shifting to lower magnetic field was observed, and the formation of a complex was confirmed.

**Example 41**

A transparent yellow solution of a complex was obtained by adding 60 g of 6FDA and γ-propiolactam to 240 g of caprolactone and heating 3 hours in an inert atmosphere. According to the nuclear magnetic resonance spectrum measured after the solution was treated by the same process as in Example 4, the same peak shifting to lower magnetic field as shown in Figs. 2 and 3 was observed, and the formation of a complex was confirmed.

**Comparative Example 11**

A brown solution was obtained by adding 60 g of 6FDA and ε -caprolactam to 240 g of NMP and heating 36 hours at 180 to 200 °C in an inert atmosphere. According to the nuclear magnetic resonance spectrum of the solution, the formation of any complex was not observed, but the quantitative formation of a bisimide compound was confirmed. The time depending change of the yield of the product was measured with a liquid chromatographic method, and it was revealed that the reaction went on very slowly, and the formation of the bisimide carboxylic acid was scarcely observed in a reaction time of 1-3 hours.

**Example 42**

A mixture of 6FDA and a complex comprising 6FDA and DMSO was dissolved in DMSO containing water, and the change of the solution was measured with the nuclear magnetic resonance spectrum. The peaks of 6FDA, which were observed at the time of the dissolution was disappeared after 18 hours, and hydrolysis of 6FDA was confirmed. On the other hand, the peak of the complex was observed without any change. With this observation, it was confirmed that the formation of a complex occured lowering of hydrolyzability and giving a significant stabilization of 6FDA.

**Claims**

1. Carboxylic acid anhydride complexes of the general formula I,

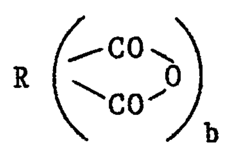 · cB (I),

wherein

R is the organic residue of the respective carboxylic acid anhydride,

B is an organic or inorganic electron donor compound forming a coordinate bond with an anhydride group, having a donor number of at least 20 and being free from active hydrogen atoms in its molecule,

b is an integer $\geqq 1$,

and

c is an integer $0 < c \leqq b$.

2. Carboxylic acid dianhydride complexes of the general formula II,

$$Ar \cdot aB \quad (II),$$

wherein

Ar is an organic carboxylic acid dianhydride,

B is an organic or inorganic electron donor compound forming a coordinate bond with an anhydride group and having a donor number of at least 20 and preferably at least 25 and being free from active hydrogen atoms in its molecule,

and

a is defined as $1 \leqq a \leqq 2$.

3. Complexes according to claim 1 or 2, of the general formula III,

$$Ar' \cdot a'B \qquad (III),$$

wherein

Ar' is an oligomeric reaction product of an organic carboxylic acid dianhydride with an organic diamine, having at least one carboxylic acid anhydride group at an end of the molecule,

B is as defined in claim 1 or 2,

and

a' is defined as $a' \geq 1$.

4. Complexes according to one of claims 1 to 3 of the general formula IV,

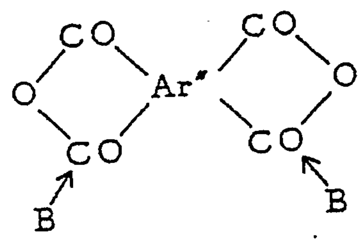

(IV),

wherein

Ar" is a residual group of a carboxylic acid dianhydride,

and

B is defined as in claim 1 or 2.

5. Complexes according to one of claims 1 to 4 of the general formula V,

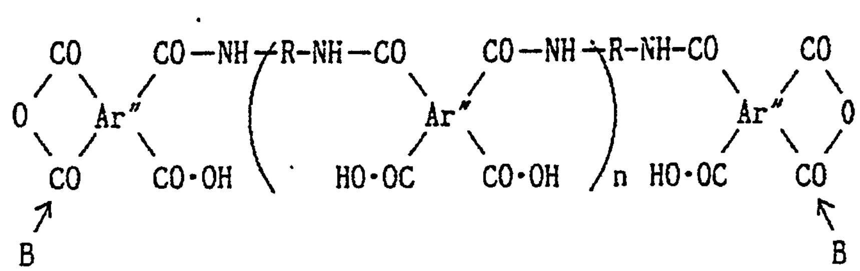

(V),

wherein

Ar" is a residual group of a carboxylic acid dianhydride,

R is an organic residual group of a diamine,

and

n is zero or an integer of at most 20.

6. A method for preparing the carboxylic acid anhydride complexes according to one of claims 1 to 5, characterized by contacting a compound comprising one or more carboxylic acid anhydride groups, particularly of the general structure

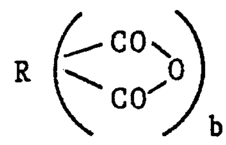

as defined above, and preferably a carboxylic acid dianhydride Ar or a compound of the general formula

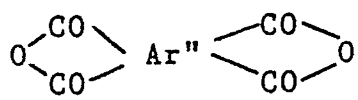

with an electron donor compound B,
where R, b, Ar, Ar" and B and the respective relative amounts of the components are defined as in the preceding claims.

**7.** The method according to claim 6, characterized in that the carboxylic acid anhydride/dianhydride is brought into contact with the electron donor compound B in the gaseous state or in the vapor phase.

**8.** The method according to claim 6, characterized in that the carboxylic acid anhydride/dianhydride is dispersed in a solvent in which it is insoluble, but the respective complex is soluble, and the elctron donor compound B is added thereto under heating.

**9.** Liquid compositions, comprising one or more complexes according to one of claims 1 to 5 and a solvent for the complex(es).

**10.** Resin compositions, comprising one or more complexes according to one of claims 1 to 5 and one or more other resins, particularly thermosetting resins.

**11.** Use of the complexes according to one of claims 1 to 5 and of the compositions according to claims 9 and 10 for producing polyimide resins and as curing agents for synthetic resins, particularly epoxy resins, and for stabilising carboxylic acid anhydrides.

FIG. 1

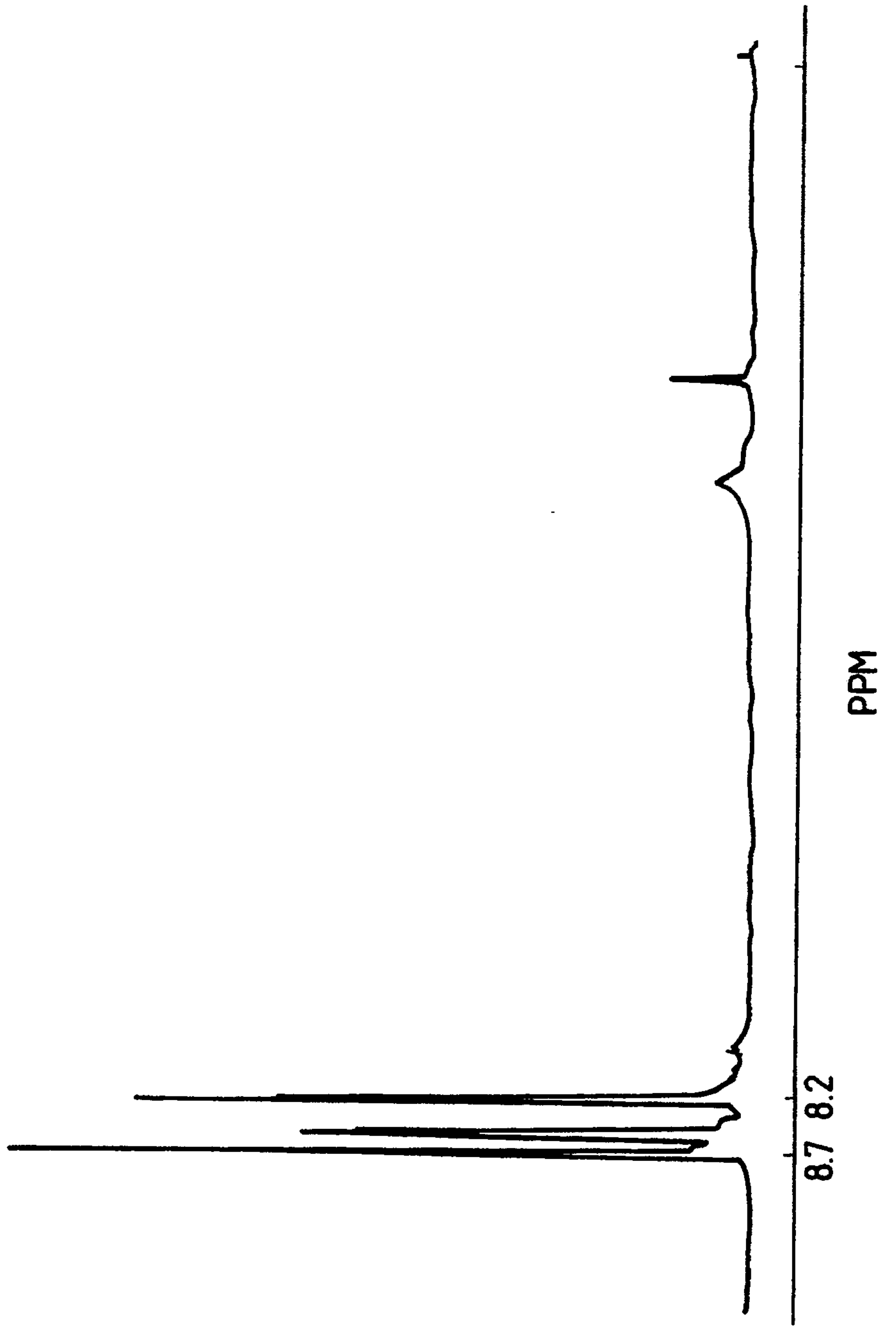

FIG. 2

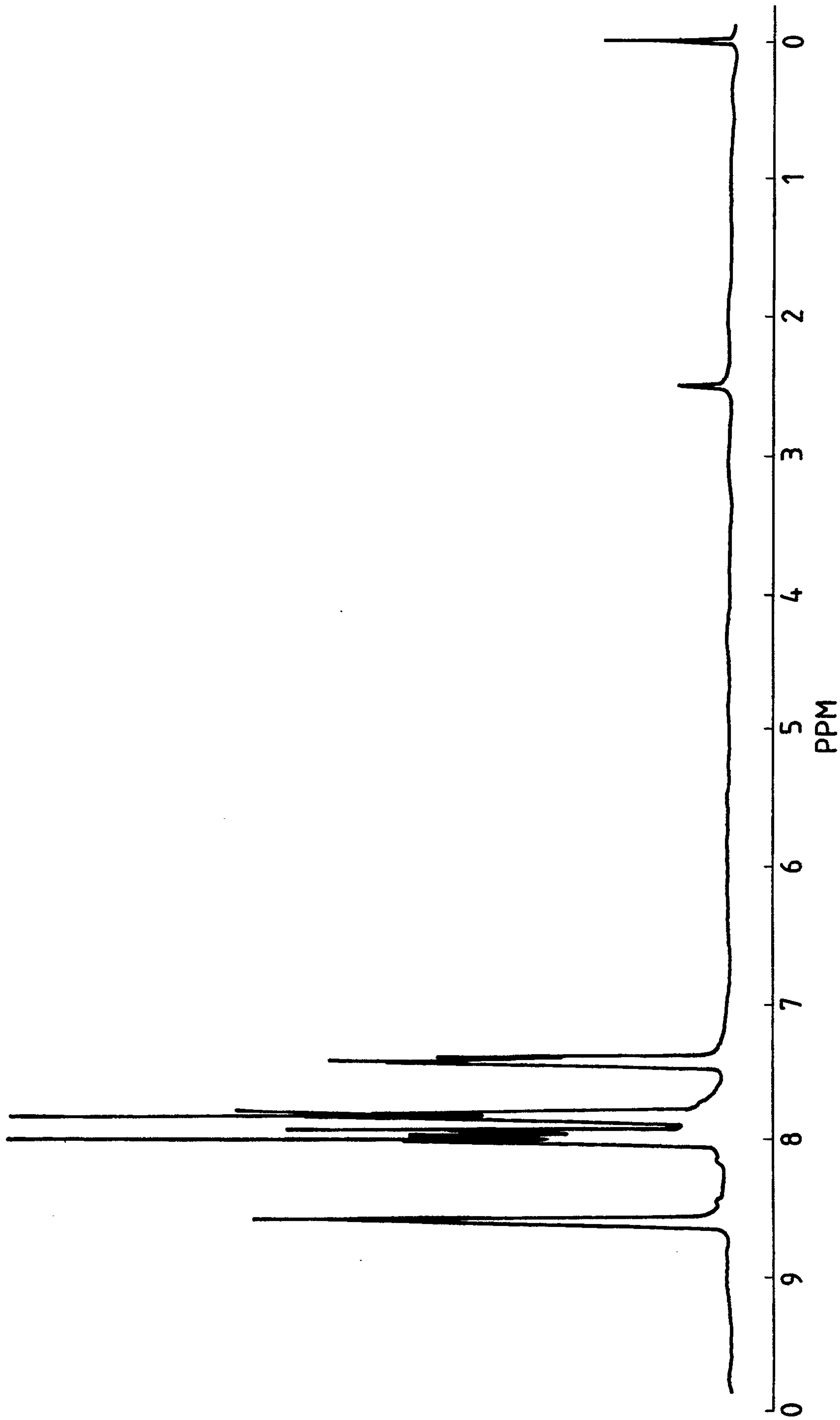

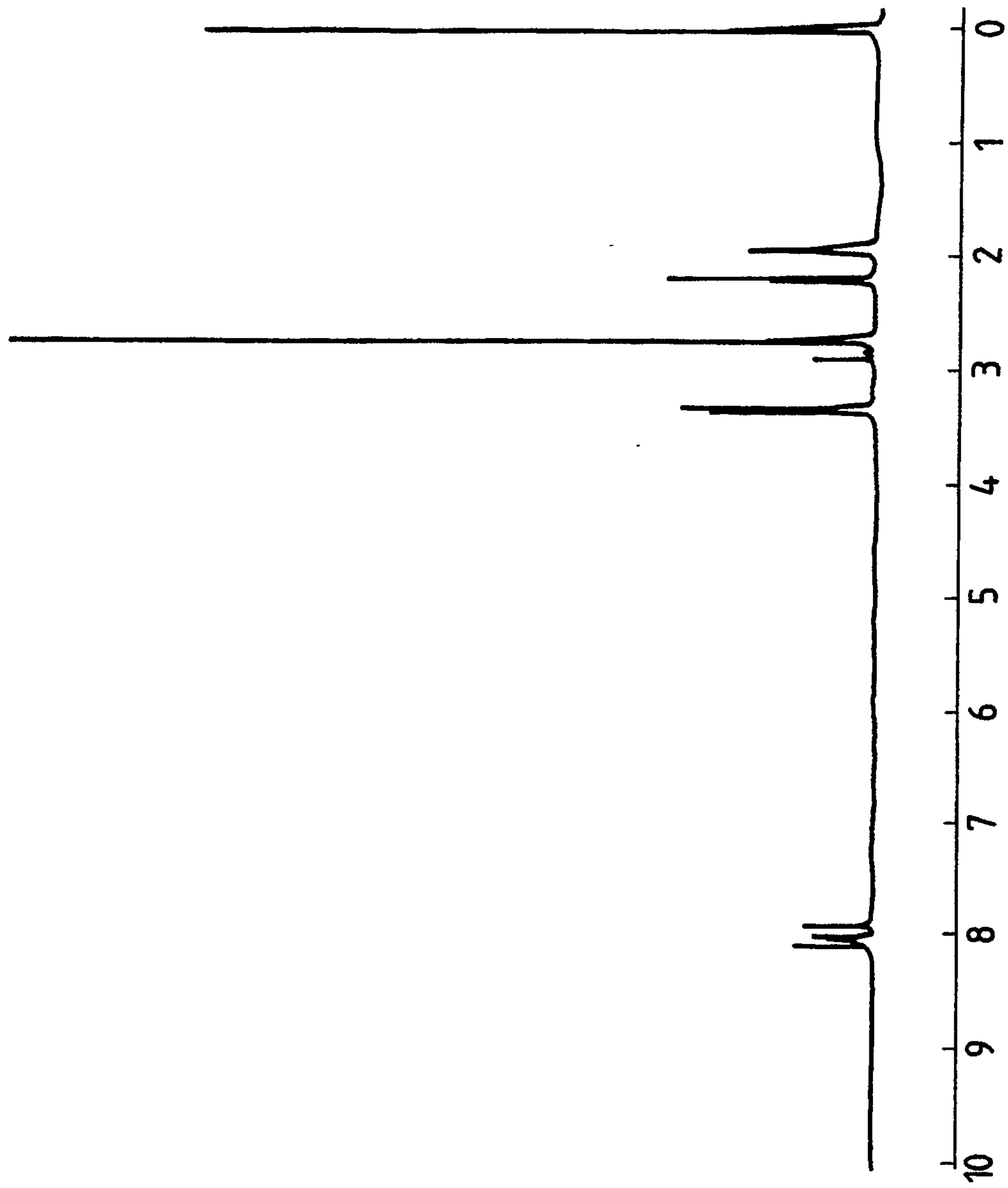


FIG. 3

FIG. 4

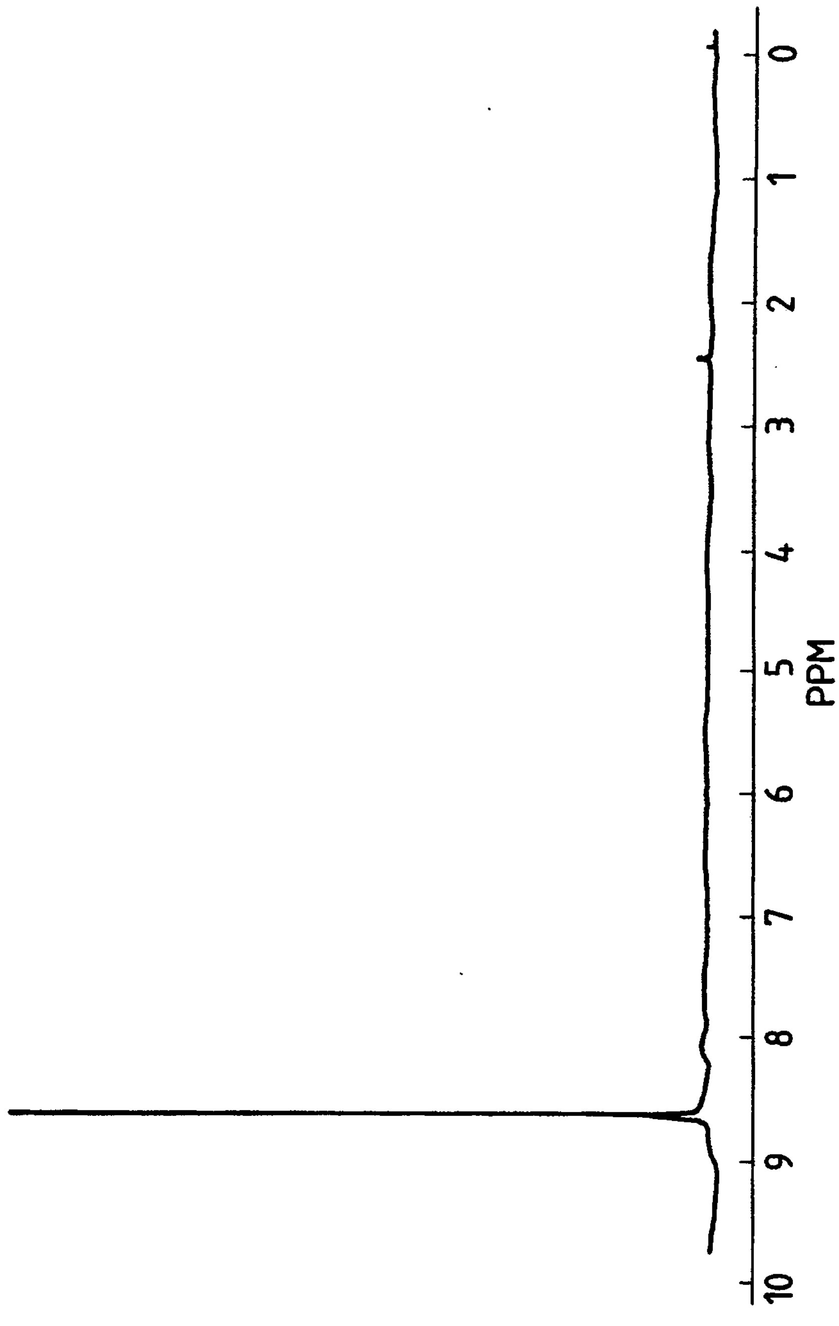

FIG. 5

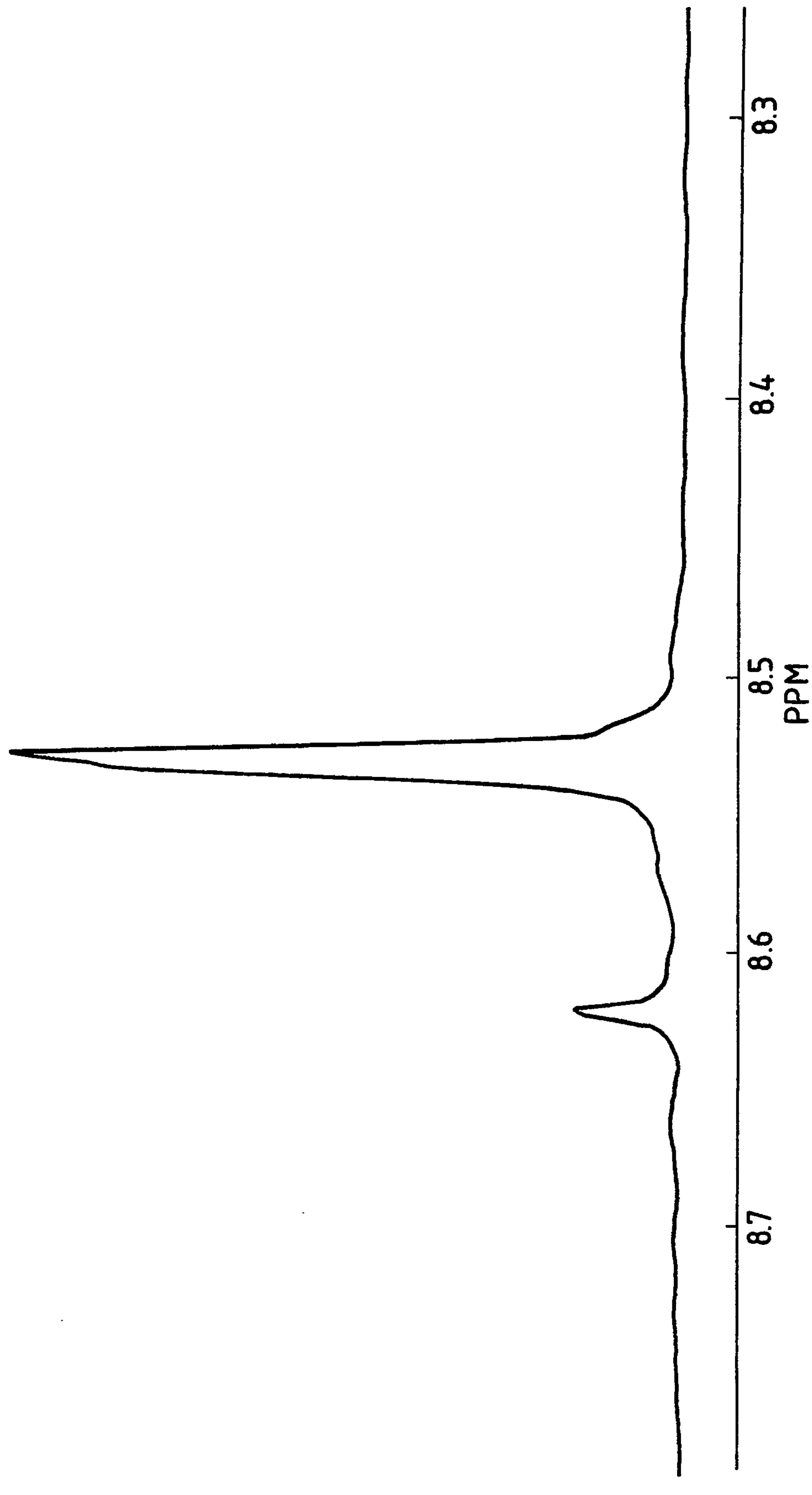

FIG. 6

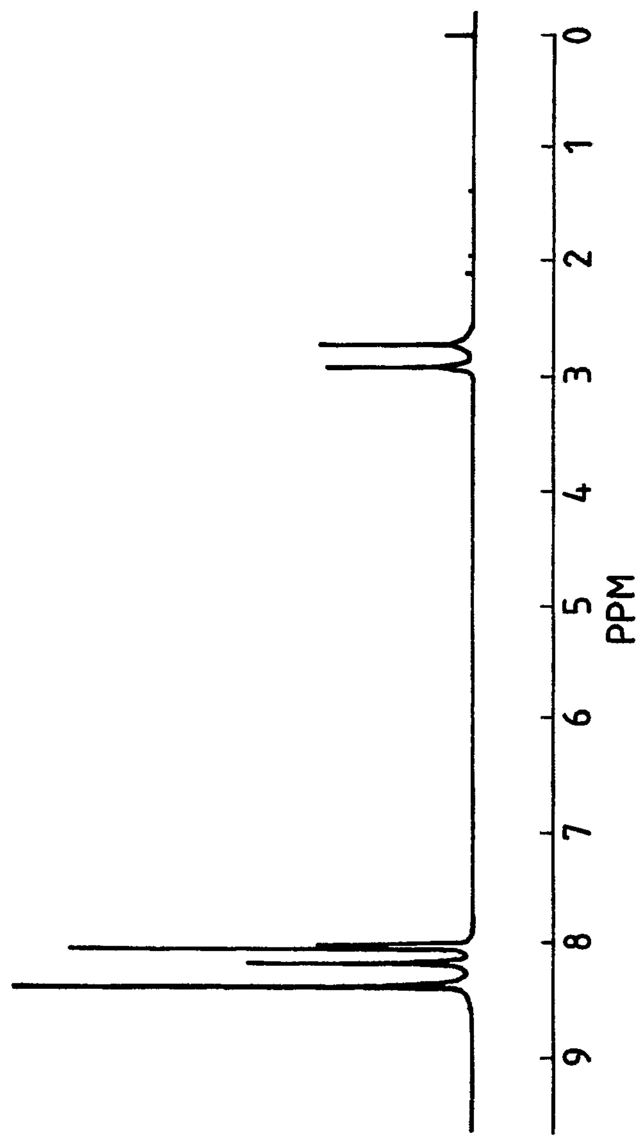

FIG. 7

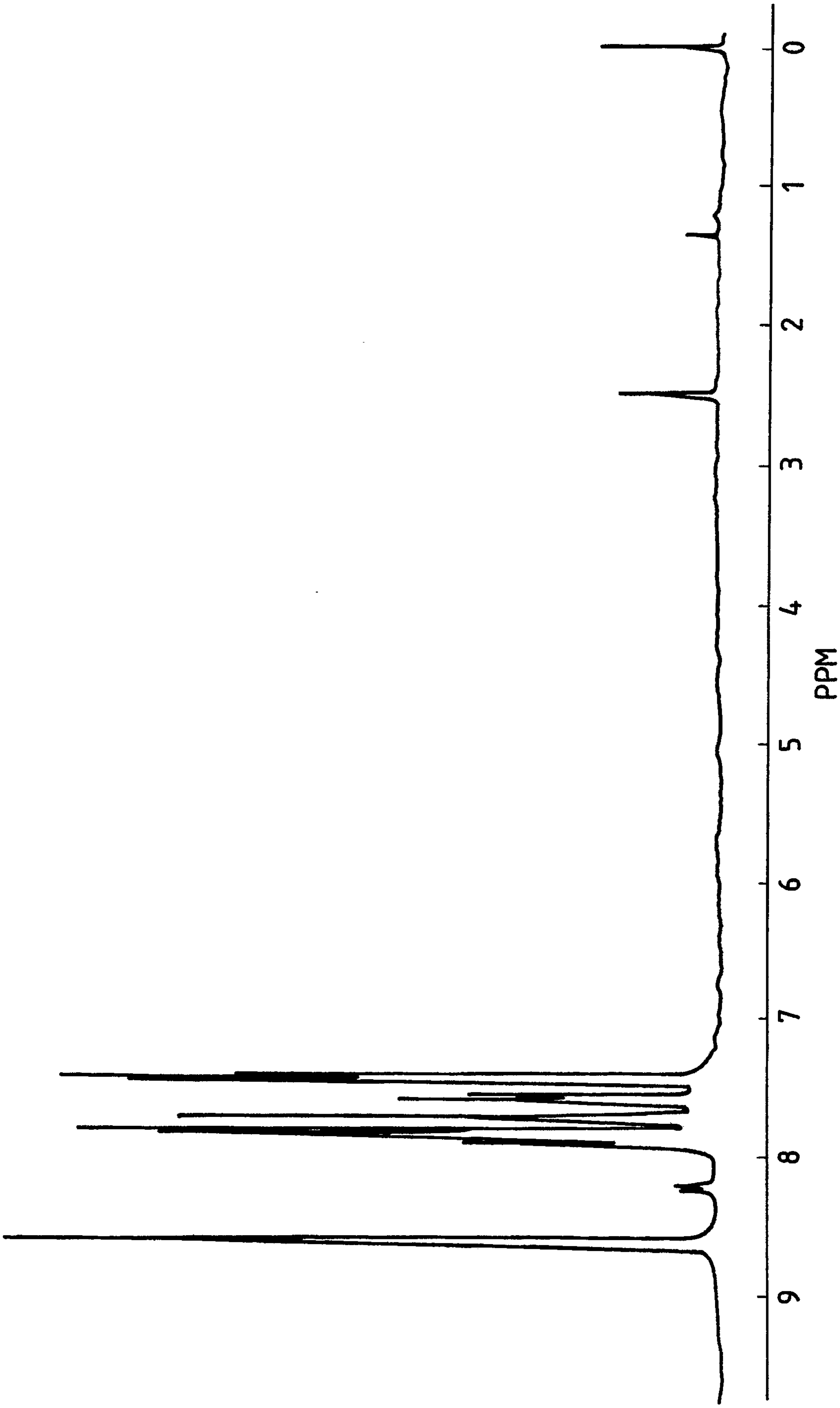

FIG. 8

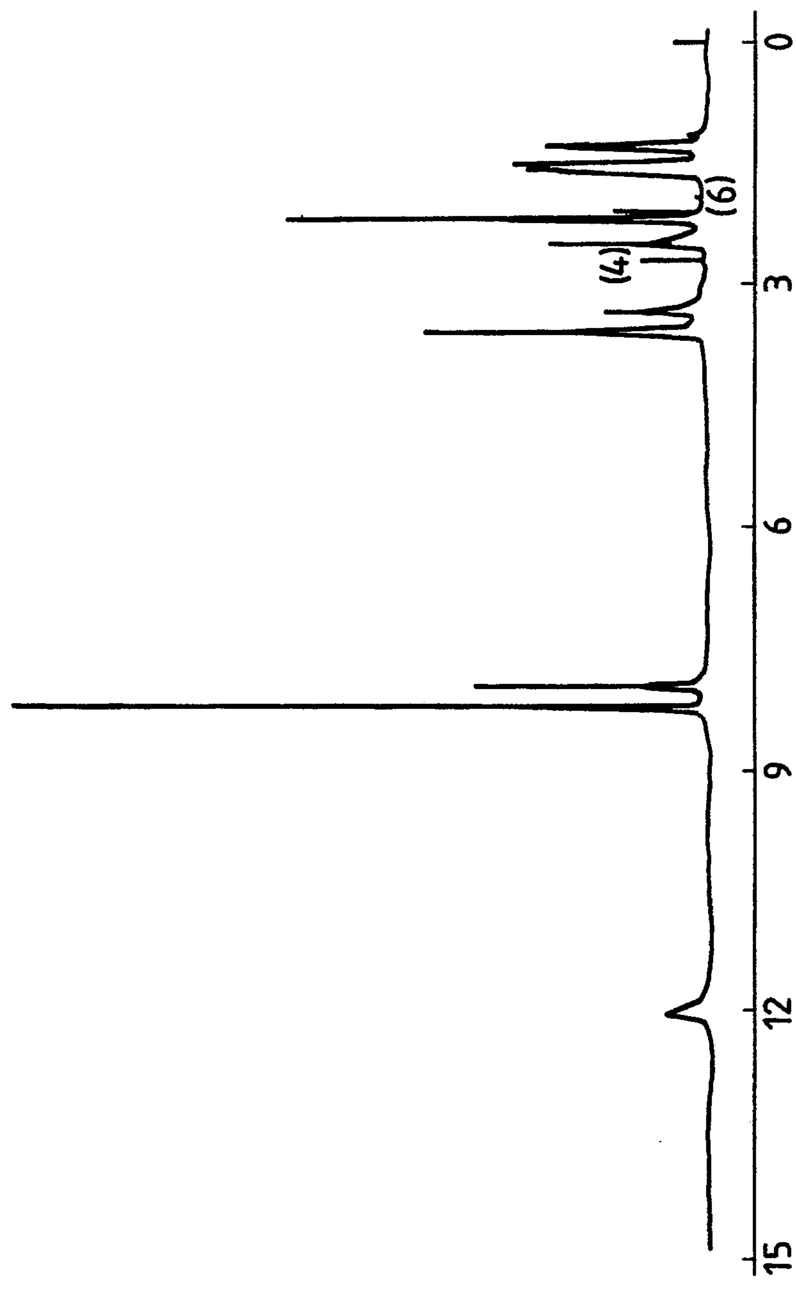

FIG. 9(a)

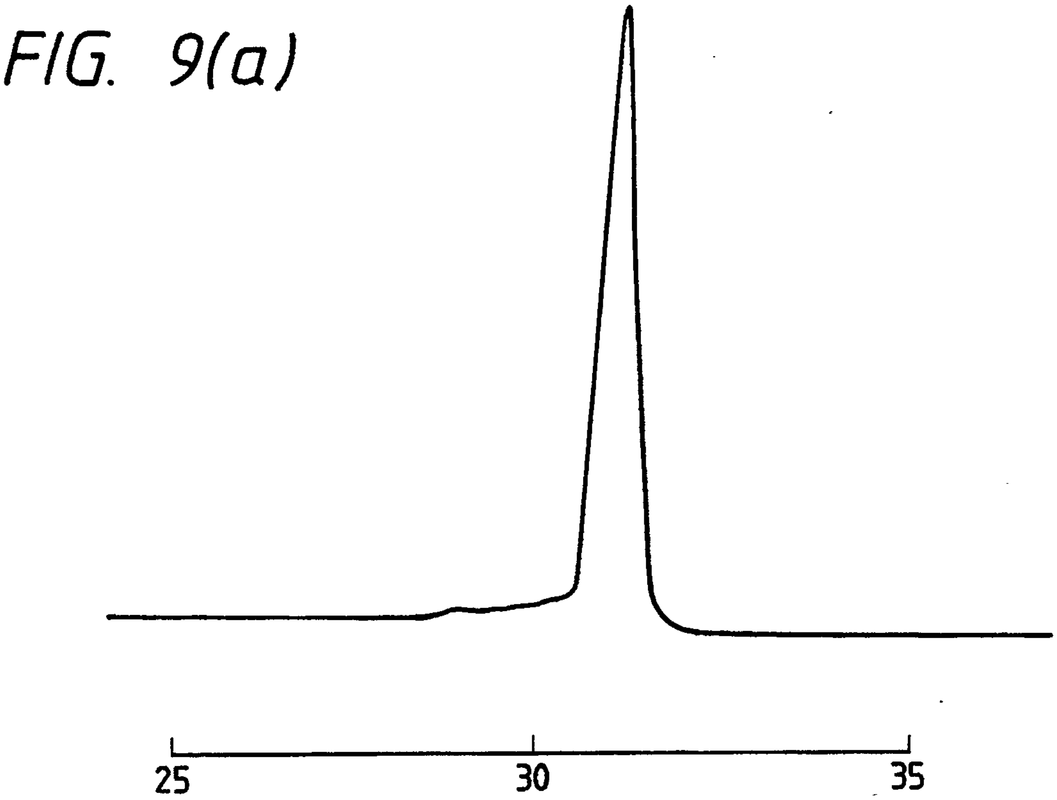


FIG. 9(b)

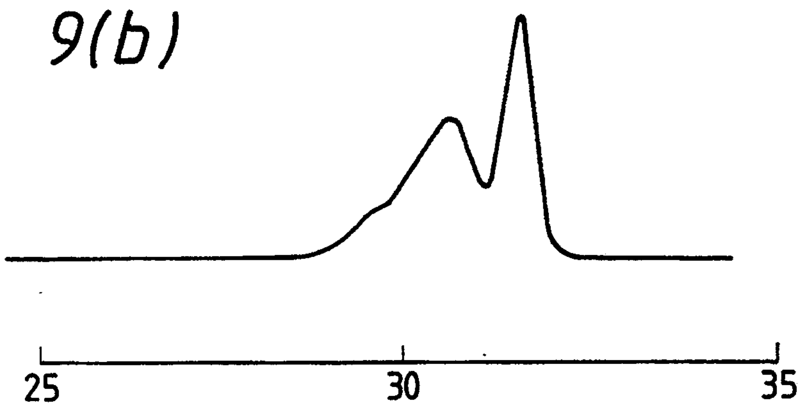


FIG. 9(c)

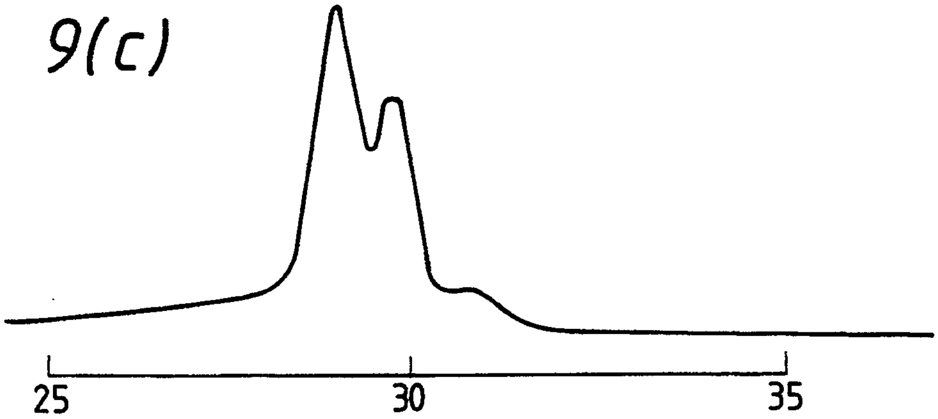

FIG. 10

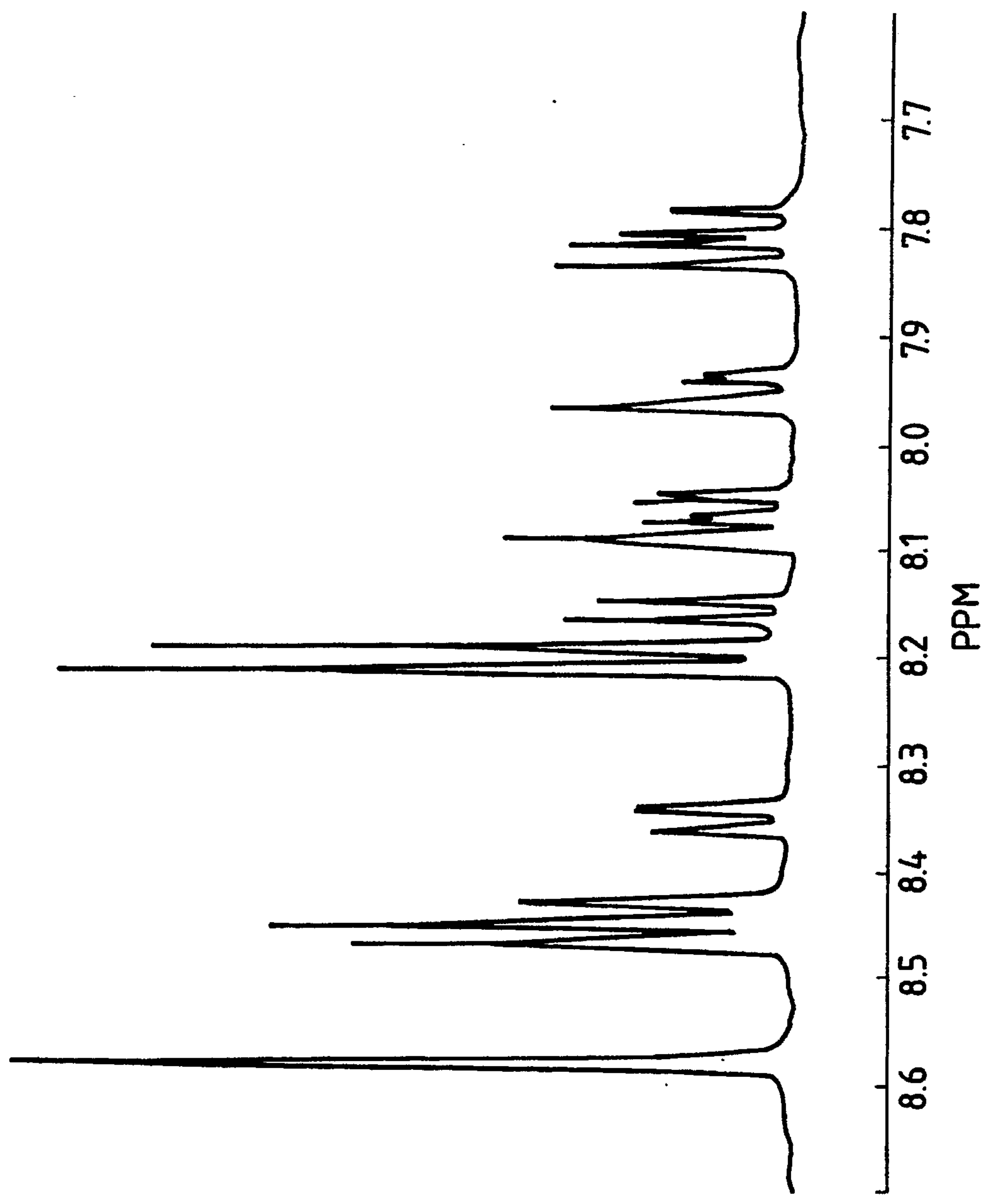

FIG. 11

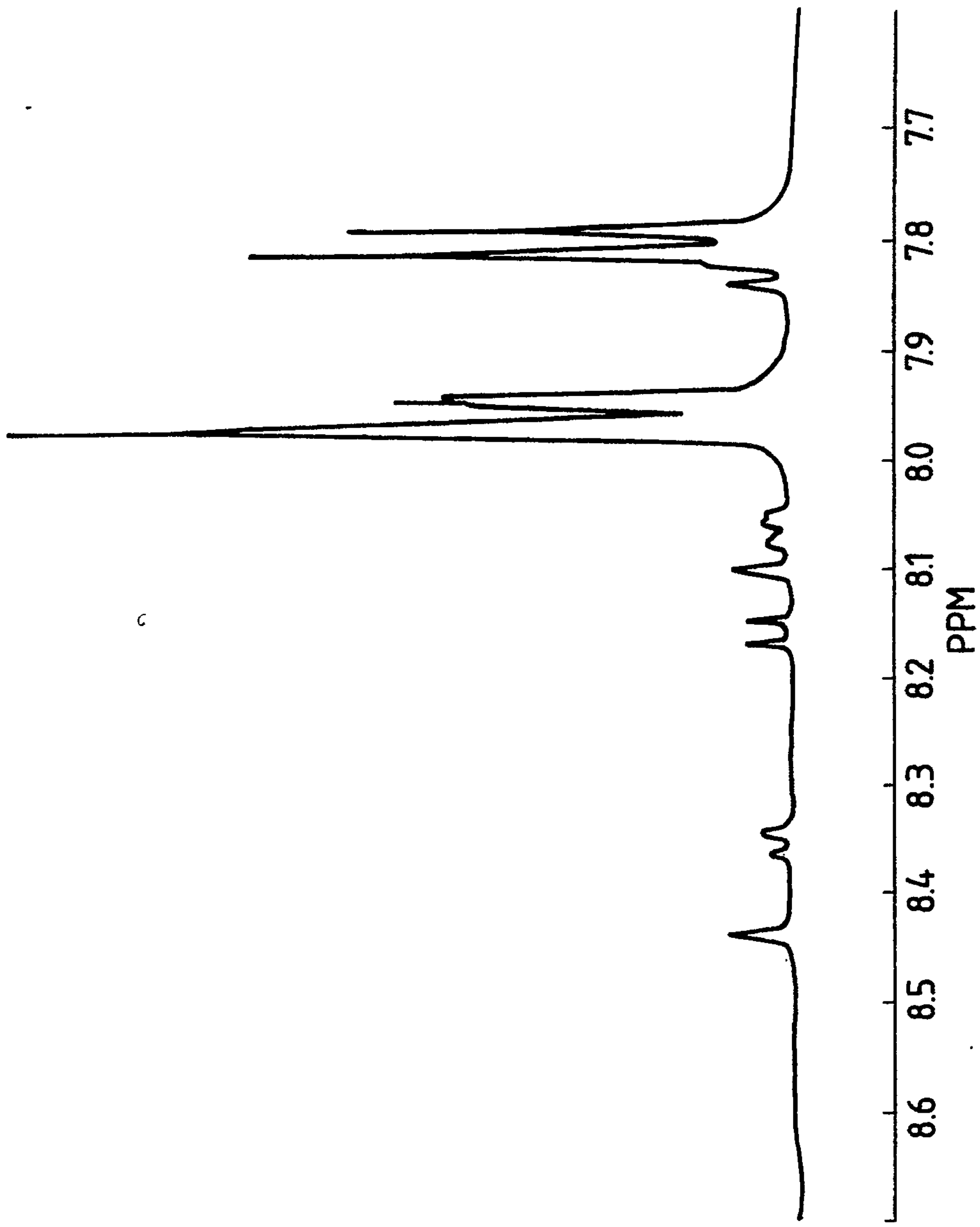

FIG. 12

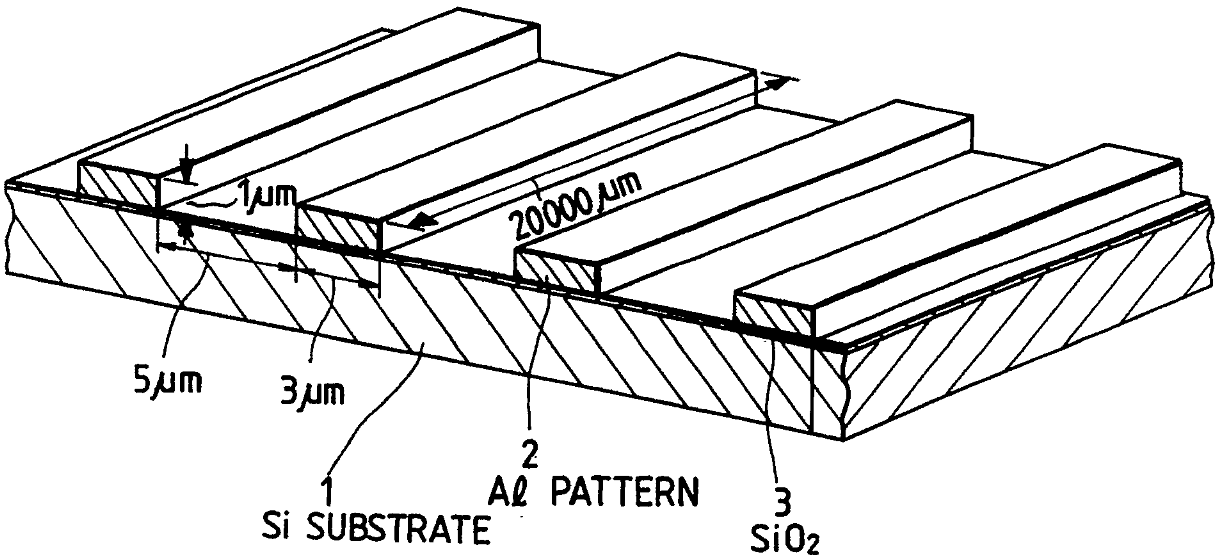


FIG. 13